# EUROPEAN PATENT APPLICATION

(11) **EP 2 100 588 A1**
(43) Date of publication of application: **16.09.2009**
(21) Application number: 07859893.5
(22) Date of filing: 20.12.2007
(51) Int. Cl.: A61K 8/49, A61K 31/4245, A61P 17/14, A61Q 7/00, C07D 413/04

(54) **PROPHYLACTIC OR THERAPEUTIC AGENT FOR ALOPECIA**

(30) Priority: 20.12.2006 JP 2006343254
(71) Applicant: Taisho Pharmaceutical Co. Ltd., Tokyo 170-8633 (JP)
(72) Inventor: NAKAO, Akiko, Tokyo 170-8633 (JP); UEMATSU, Natsuko, Tokyo 170-8633 (JP); TAKAHASHI, Akiko, Tokyo 170-8633 (JP); YOKOTA, Shin-ichi, Tokyo 170-8633 (JP)
(74) Representative: Siegert, Georg
(86) International application number: PCT/JP2007/074496
(87) International publication number: WO 2008/075735

(57) **Abstract**

Disclosed is a novel therapeutic agent which is useful for the prevention, or treatment of alopecia. Specifically disclosed is a prophylactic or therapeutic agent for alopecia, which comprises 1-[2-((2S)-2-{5-[(3,4-dimethoxyphenoxy)methyl]-1,2,4-oxadiazol-3-yl}pyrrolidin-1-yl)-1,1-difluoro-2-oxoethyl]-3,3,5,5-tetramethylcyclohexanol as an active ingredient.

## Description

### Technical Field

This invention relates to a prophylactic or therapeutic agent for alopecia, more specifically, to a prophylactic or therapeutic agent for alopecia containing a specific aryloxymethyloxadiazole derivative as an active ingredient.

### Background Art

There are various types of alopecia such as androgenetic alopecia, alopecia senilis, alopecia areata, and alopecia of postmenopausal women. Many of them are not life-threatening but involve mental distress due to problems in appearance, and there is a demand for an excellent prophylactic or therapeutic agent for alopecia.

Hairs are renewed via steps of anagen, catagen, and telogen (hair cycle). One cycle of the hair cycle ordinarily requires a time period of 2 to 7 years, but, growth of hair is stopped before full growth of the hair when the time period is shortened due to a certain abnormality. As a result, a reduction in density of hairs due to an increase in number of lost hairs and a reduction in thickness of each of hairs are observed. Examples of factors that disturb the rhythm of the hair cycle include androgen such as testosterone and dihydrotestosterone, radioactive ray, drugs such as anticancer drug, aging, stress, and the like.

Many studies using various compounds have been made for the purpose of creation of a therapeutic agent for alopecia, and it has been reported that an immunosuppressant FK506 (tacrolimus), for example, has a hair growth stimulatory effect in a plurality of animal models (Patent Document 1 and Non-Patent Document 1). The effect is confirmed not only in a model of alopecia areata which is considered to be an autoimmune disorder (Non-Patent Documents 2 and 3) but also in hair growth test using normal mice and a drug-induced alopecia model (Non-Patent Documents 4 and 5). However, FK506 has the high risk of adverse effects due to its immunosuppressive action, and there has been a demand for a safer compound that is reduced in immunosuppressive action as the therapeutic agent for alopecia.

Recently, it has been revealed that a compound bound to immunophilin FKBP12 (protein that is bound to FK506 and has a molecular weight of 12 kDa) has cytoprotective action similar to that of FK506 without immunosuppressive action, and a plurality of derivatives have been found (Patent Documents 2 to 9). It is disclosed that a part of the derivatives has the hair growth stimulatory action (Patent Document 10). However, no report regarding the hair growth stimulatory action has been made on other derivatives, and there are many unclear points about a relationship between the binding activity to immunophilin FKBP12 and the hair growth stimulatory activity.

Though aryloxymethyloxadiazole derivatives are disclosed (Patent Document 11) as the compounds bound to FKDP12, there is no report on hair growth stimulatory action of these derivatives.
Patent Document 1: Japanese Patent No. 2925285
Patent Document 2: International Publication No. WO96/40633
Patent Document 3: International Publication No. WO92/19593
Patent Document 4: International Publication No. WO00/27811
Patent Document 5: International Publication No. WO99/62511
Patent Document 6: International Publication No. WO99/45006
Patent Document 7: International Publication No. WO00/05231
Patent Document 8: International Publication No. WO01/42245
Patent Document 9: JP-A-2004-123556
Patent Document 10: International Publication No. WO98/5509
Patent Document 11: JP-A-2004-123557
Non-Patent Document 1: Yamamoto, et al., "J. Invest. Dermatol.", 102, 160-164, 1994
Non-Patent Document 2: Freyschmidt-Paul et al., "Eur. J. Dermatol.", 11, 405-409, 2001
Non-Patent Document 3: McElwee et al, "Br. J. Dermatol. ", 137, 491-497, 1997
Non-Patent Document 4: Jiang et al., "J. Invest. Dermatol.", 104, 523-525, 1995
Non-Patent Document 5: Maurer et al., "Am. J. Pathol.", 150, 1433-1441, 1997

### Disclosure of the Invention

### Problems that the Invention is to Solve

An object of the present invention is to provide a novel therapeutic agent useful for prophylaxis and treatment of alopecia.

### Means for Solving the Problems

The inventors had focused on aryloxymethyloxadiazole derivatives and searched for pharmacological action of these compounds to find that a specific compound among the derivatives has an excellent hair growth stimulatory action and is free from problematic adverse effects, and thereby have accomplished the present invention.

Specifically, the present invention provides:
(1) A prophylactic or therapeutic agent for alopecia, comprising 1-[2-((2S)-2-{5-[(3,4-dimethoxyphenoxy)methyl]-1,2,4-oxadiazol-3-yl}pyrrolidin-1-yl)-1,1-difluoro-2-oxoethyl]-3,3,5,5-tetramethylcyclohexanol as an active ingredient;
(2) A prophylactic or therapeutic method for alopecia, comprising administering 1-[2-((2S)-2-{5-[(3,4-dimethoxyphenoxy)methyl]-1,2,4-oxadiazol-3-yl}pyrrolidin-1-yl)-1,1-difluoro-2-oxoethyl]-3,3,5,5-tetramethylcyclohexanol in and amount effective for prophylaxis or treatment of alopecia to a mammal; and
(3) A use of 1-[2-((2S)-2-{5-[(3,4-dimethoxyphenoxy)methyl]-1,2,4-oxadiazol-3-yl}pyrrolidin-1-yl)-1,1-difluoro-2-oxoethyl]-3,3,5,5-tetramethylcyclohexanol for production of a prophylactic or therapeutic agent for alopecia.

### Advantages of the Invention

In the present invention, it was found that, among the compounds belonging to an aryloxymethyloxadiazole derivative that is bound to immunophilin FKBP12 to inhibit the rotamase activity, 1-[2-((2S)-2-{5-[(3,4-di-methoxyphenoxy)methyl]-1,2,4-oxadiazol-3-yl}pyrrolidin-1-yl)-1,1-difluoro-2-oxoethyl]-3,3,5,5-tetramethylcyclohexanol has excellent hair growth stimulatory action.

Since the compound scarcely has immunosuppressive action and the like, a drug formulation containing the compound is advantageously used as a prophylactic or therapeutic agent for alopecia.

### Best Mode for Carrying out the Invention

In this specification, "alopecia" means a state in which a part or whole of hairs is fallen out or lost or a state in which a part or whole of hairs becomes thinner and shorter. Alopecia includes, but not particularly limited to, androgenetic alopecia, seborrheic alopecia, alopecia senilis, alopecia areata, drug-induced alopecia caused by administration of an anticancer drug or the like, cicatricial alopecia, postpartum alopecia that occurs after delivery. Alopecia is generally attributable to a failure of hair cycle and triggered by shortening of duration of anagen which is caused by stoppage of cell proliferation.

Also, "hair cycle" means a growth cycle of hairs and includes 3 stages of (1) anagen (a period in which hair follicle cell division occurs repeatedly so that hairs grow actively, which continues for 2 to 6 years for scalp hair), (2) catagen (a period in which hair growth is decreased so that follicle is shrunk, which continues for 1 to 2 weeks for scalp hair), and (3) telogen (a period in which follicle is completely regressed and in cessation, which continues for 3 to 4 months for scalp hair). Generally, 80% to 90% of hair is in anagen; less than 1% of hair is in catagen; and the rest is in telogen. Abnormality occurs in the hair cycle in alopecia, and, particularly in androgenetic alopecia, the period of anagen is shortened so that anagen shifts to catagen/telogen before hair grows to thick terminal hair, thereby causing an increase in proportion of telogen hairs and a change from terminal hairs to thin vellus hairs.

Further, "prophylactic or therapeutic agent for alopecia" includes those having at least one of (1) induction of anagen from telogen (hair growth induction), (2) stimulation of hair growth, (3) extension of anagen, and (4) inhibition, retardation, or decrease of hair loss, and those having a plurality actions among the above actions are desired.

The active ingredient of the prophylactic or therapeutic agent for alopecia of the present invention, which is 1-[2-((2S)-2-{5-[(3,4-dimethoxyphenoxy)methyl]-1,2,4-oxadiazol-3-yl}pyrrolidin-1-yl)-1,1-difluoro-2-oxoethyl]-3,3,5,5-tetramethylcyclohexanol (hereinafter referred to as "compound of the present invention"), is the compound represented by the following formula:

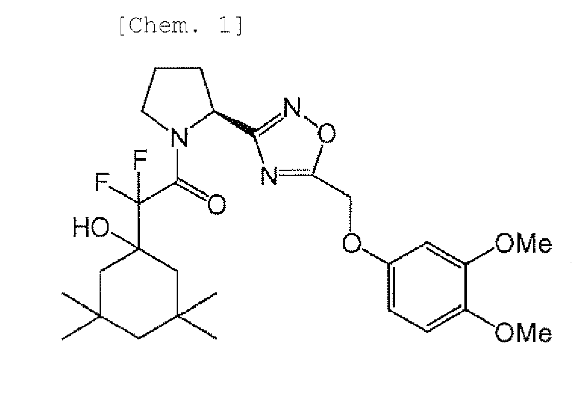

The compound is identical to the compound disclosed in Example 1 of JP-A-2004-123557 and obtainable from the method disclosed in the publication or methods described in Examples described later in this specification. Also, the FKBP12 rotamase inhibitory activity of the compound of the present invention is disclosed in the publication.

The compound of the present invention can exist as various solvates. Also, in view of applicability to drugs, hydrate of the compound is useful.

It is possible to administer the prophylactic or therapeutic agent for alopecia in the present invention via an oral route, a parenteral route or a local route. In view of points such as direct administration to affected area, easy administration, reduction in probability of systemic adverse effect, and the like, a preferred dosage form is an external preparation. Also, since the compound of the present invention has a low probability of systemic adverse effect, it is possible to use the compound as an oral preparation.

It is possible to prepare the prophylactic or therapeutic agent for alopecia of the present invention by using the above-described compound of the invention, a known carrier, a diluting agent, and the like as required and by forming into an appropriate pharmaceutical composition. More specifically, the prophylactic or therapeutic agent is usable as the oral preparation such as a tablet, a powder, a dispersant, a granule, a liquid, a capsule, a dry syrup, and a jelly as well as the external preparation such as a liquid, a lotion, an ointment, a pack, and a cream.

In the case of using the prophylactic or therapeutic agent for alopecia of the present invention as the oral preparation, it is possible to add other known additives such as vitamins, amino acids, herbal medicines, natural substances, vehicles, pH adjusters, algefacients, suspending agents, thickening agents, solubilizing agents, disintegrating agents, binding agents, lubricants, antioxidants, coating agents, coloring agents, flavoring agents, surfactants, plasticizers, and fragrances as required within qualitative and quantitative ranges that do not impair the effects of the invention.

In the case of using the prophylactic or therapeutic agent for alopecia of the present invention as the external preparation, it is possible to add other known additives such as solvents including 1,3-butyleneglycol, ethanol, methanol, purified water, and the like, preservatives including paraben and the like, disinfectants including hinokitiol and the like, oils such as white petrolatum, squalane, paraffin, and the like, esters such as cetyl 2-ethylhexanoate, cetyl caprate, glyceryl monooleate, and the like, silicon derivatives including a silicon resin, silicon oil, and the like, surfactants including polyoxyethylene hydrogenated castor oil and the like, gelling agents including a carboxyvinyl polymer, polyvinyl alcohol, and the like, pH adjusters, antioxidants, and coloring agents as required within qualitative and quantitative ranges that do not impair the effects of the invention.

An administration amount of the prophylactic or therapeutic agent for alopecia of the present invention to be produced as described above can be adjusted according to body weight, age, sex, and the like of a patient. More specifically, in the case of using the external preparation, the preparation contains the compound of the present invention in a concentration of 0.0001% to 20% and can be administered once to several times per day. An application amount with respect to hair may be about 0.00001 to 4 mg/cm², preferably about 0.01 to 1 mg/cm².

In the case of using the oral preparation, 1 to 100 mg/kg of the compound of the present invention is used per day for an adult, and the preparation is administered once to several times per day.

It is possible to use an active ingredient of another prophylactic or therapeutic agent for alopecia in combination with the compound of the present invention. Examples of the drug to be used in combination include, but not limited to, minoxidil, finasteride, and the like. Also, combined use with drugs such as another hair growth agent/hair restorer, vasodilative agents, antiandrogenic agents, cyclosporine derivatives, antibacterial agents, anti-inflammatory agents, thyroid hormone derivatives, prostaglandin agonists or antagonists, retinoids, and triterpenes is possible.

### Examples

Hereinafter, the present invention will be described in more details by using synthesis examples, preparation examples, test example, and the like, but the invention is not limited by these examples at all.

### Synthesis Example

Though it is possible to produce the compound of the present invention in accordance with the production method disclosed in JP-A-2004-123557, the compound is also obtainable by a method described below.

Synthesis of 1-[2-((2S)-2-{5-[(3,4-dimethoxyphenoxy)methyl]-1,2,4-oxadiazol-3-yl}pyrrolidin-1-yl)-1,1-difluoro-2-oxoethyl]-3,3,5,5-tetramethylcyclohexanol:

### (1) t-butyl(2S)-2-[(Z)-amino(hydroxyimino)methyl]pyrrolidine-1-carboxylate

Hydroxylamine hydrochloride (106 g, 1.53 mol) and sodium carbonate (162 g, 1.53 mol) were added to a mixed solution of t-butyl(2S)-2-cyanopyrrolidine-1-carboxylate (150 g, 0.76 mol), water (700 ml), and methanol (700 ml), followed by heating with stirring at 100°C for 5 hours. After the reaction, methanol was distilled away under a reduced pressure, and water (1500 ml) was added, followed by filtration and drying of a precipitate, thereby obtaining a colorless crystal (88.4 g). Chloroform was added to the filtrate, followed by washing with saturated saline. After drying with magnesium sulfate, the solvent was distilled away under a reduced pressure to obtain a colorless crystal (77.4 g). The total amount was 166 g.

### ¹H-NMR (200 MHz, chloroform-D)

δ ppm 1.46 (s, 9 H), 1.75 - 2.43 (m, 4 H), 3.24 - 3.56 (m, 2 H), 4.12 - 4.72 (m, 2 H), 5.30 (m, 1 H)

### (2) (3,4-dimethoxyphonoxy)acetic acid

Potassium carbonate (405 g, 2.93 mol) and ethyl bromoacetate (391 g. 2.34 mol) were added to an acetone (3000 ml) solution of 3,4-dimethoxyphenol (300 g, 1.95 mol), followed by stirring at 60°C for 4 hours. Subsequently, ethyl bromoacetate (33.1 g, 0.198 mol) was added, followed by stirring at 65°C for 1.5 hours. Further, ethyl bromoacetate (33.1 g, 0.198 mol) was added, followed by stirring at 65°C for 2.5 hours. Ethyl bromoacetate (33.1 g, 0.198 mol) was added, followed by stirring at 65°C for 2 hours.

The reaction liquid was returned to a room temperature, and potassium carbonate was eliminated by filtration, followed by distilling away the solvent and excessive ethyl bromoacetate under a reduced pressure, thereby obtaining a brown oily substance (485 g). An aqueous solution (550 ml) of sodium hydroxide (195 g) was added to an ethanol (1100 ml) solution of the thus-obtained ethyl (3,4-dimethoxyphenoxy)acetate crude product, followed by stirring at a room temperature for one hour. After adding a concentrated hydrochloric acid (440 ml), the solvent was distilled away.

A precipitate was filtrated and washed with water to obtain a light brown solid matter (335 g). Chloroform was added to the filtrate, followed by washing with saturated saline. After drying with magnesium sulfate, the solvent was distilled away under a reduced pressure. Recrystallization from ethyl acetate/hexane was conducted to obtain a light brown powder (33.1 g). The total amount was 368 g.

¹H-NMR (200 MHz, chloroform-D)
δ ppm 3.84 (s, 3 H), 3.86 (s, 3 H), 4.64 (s, 2 H), 6.37 (dd, J=8.57, 2.86 Hz, 1 H), 6.61 (d, J=2.64 Hz, 1 H), 6.77 (d, J=8.79 Hz, 1 H), 10.45 (br.s, 1 H)

### (3) t-butyl(2S)-2-{5-[3,4-dimethoxyphenoxy)methyl]-1,2,4-oxadiazol-3-yl}pyrrolidine-1-carboxylate

Triethylamine (422 g, 4.17 mol) was added to a chloroform solution (2000 ml) of t-butyl(2S)-2-[(Z)-amino(hydroxyimino)methyl]pyrrolidine-1-carboxylate (319 g, 1.39 mol), (3,4-dimethoxyphenoxy)acetic acid (369 g, 1. 74 mol), 1-hydroxybenzotriazole monohydrate (245 g, 1.81 mol), and 1-ethyl-3-(3-dimethylaminopropyl)carbodiimide hydrochloride (347 g, 1.81 mol) for one hour, followed by stirring at a room temperature for 19 hours. After distilling away the solvent, ethyl acetate was added, followed by washing with water, a saturated aqueous sodium hydrogencarbonate solution, and saturated saline in this order. After drying with magnesium sulfate, the solvent was distilled away under a reduced pressure, thereby obtaining a crude product as a brown oily substance (389 g).

The crude product (389 g) was heated to reflux in xylene (1000 ml) for 6 hours. After distilling away the solvent, ethyl acetate was added, followed by washing with 1 mol/L-hydrochloric acid, a saturated aqueous sodium hydrogencarbonate solution, and saturated saline in this order. After drying with magnesium sulfate, the solvent was distilled away under a reduced pressure, thereby obtaining a brown oily substance (257 g).

### ¹H-NMR (200 MHz, chloroform-D)

δ ppm 1.04 - 2.43 (m, 13 H), 3.34 - 3.71 (m, 2 H), 3.83 (s, 3 H), 3.86 (s, 3 H), 4.93 - 5.14 (m, 1 H), 5.21 (s, 2 H), 6.46 (dd, J=8. 79, 2.64 Hz, 1 H), 6.64 (d, J=2. 64 Hz, 1 H), 6. 77 (d, J=8.79 Hz, 1 H)

### (4) 5-[(3,4-dimethoxyphenoxy)methyl]-3-[(2S)-pyrrolidin-2-yl]-1,2,4-oxadiazole

4 mol/L-hydrochloric acid/ethyl acetate (750 ml) was added to an ethyl acetate (750 ml) solution of t-butyl(2S)-2-{5-[(3,4-dimethoxyphenoxy)methyl]-1,2,4-oxadiazol-3-yl}pyrrolidine-1-carboxylate (311 g, 0.77 mol), followed by stirring at a room temperature for 4 days. After adding water, washing with ethyl acetate was performed, and neutralization was performed by using a 20% NaOH aqueous solution. Ethyl acetate was added, followed by washing with water. After drying with magnesium sulfate, the solvent was distilled away under a reduced pressure, thereby obtaining a brown oily substance (186 g).

### ¹H-NMR (200 MHz, chloroform-D)

δ ppm 1.79 - 2.31 (m, 4 H), 2.98 - 3.22 (m, 2 H), 3.84 (s, 3 H) , 3.86 (s, 3 H), 4.39 - 4.46 (m, 1 H), 5.22 (s, 2 H), 6.47 (dd, J=2.64 , 8.79 Hz, 1 H), 6. 64 (d, J-2.64 Hz, 1H), 6.77 (d, J=8.79 Hz, 1H)

### (5) difluoro(1-hydroxy-3,3,5,5-tetramethylcyclohexyl)acetic acid

Under a nitrogen gas stream, 1,2 -dibromoethane and trimethylsilylchloride (3 ml each) were added to a tetrahydrofuran (500 ml) solution of a zinc powder (59.5 g, 0.91 mol), followed by activation. Subsequently, a tetrahydrofuran (200 ml) mixture solution of 3,3,5,5-tetramethylcyclohexanone (108 g, 0.70 mol) and ethyl bromodifluoroacetate (157 g, 0.77 mol) was added by dropping, followed by heating to reflux for 3.5 hours. After the reaction, a saturated aqueous ammonium chloride solution was added, and the non-reacted zinc powder was eliminated by filtration. Ethyl acetate was added, followed by washing with water and saturated saline. After drying with magnesium sulfate, the solvent was distilled away under a reduced pressure, thereby obtaining a yellow oily substance (201 g) of ethyl difluoro(1-hydroxy-3,3,5,5-tetramethylcyclohexyl)acetate.

An aqueous solution (300 ml) of sodium hydroxide (42.0 g, 1.05 mol) was added to an ethanol (800 ml) solution of the thus-obtained compound (201 g), followed by stirring at a room temperature for 3.5 hours. After distilling away the solvent, 6 mol/L-hydrochloric acid was added, and ethyl acetate was further added, followed by washing with saturated saline. After drying with magnesium sulfate, the solvent was distilled away under a reduced pressure, thereby obtaining a colorless solid matter (169 g).

### ¹H-NMR (200 MHz, chloroform-D)

δ ppm 0.97 (s, 6 H), 1.10 - 1.71 (m, 6 H), 1.23 (s, 6 H), 5.55 (br.s, 1 H)

### (6) 1-[2-(2S)-2-{5-[(3,4-dimethoxyphenoxy)methyl]-1,2,4-oxadiazol-3-yl}pyrrolidin-1-yl)-1,1-difluoro-2-oxoethyl]-3,3,5,5-tetramethylcyclohexanol

Difluoro(1-hydroxy-3,3,5,5-tetramethylcyclohexyl)acetic acid (177 g, 0.71 mol), 1-hydroxybenzotriazole monohydrate (104 g, 0.77 mol), and 1-ethyl-3-(3-dimethylaminopropyl)carbodiimide hydrochloride (148 g, 0.77 mol) were added to a chloroform (900 ml) solution of 5-[(3,4-dimethoxyphenoxy)methyl]-3-[(2S)-pyrrolidin-2-yl]-1,2,4-oxadiazole (180 g, 0.59 mol), followed by stirring at a room temperature for 4 hours. Difluoro(1-hydroxy-3,3,5,5-tetramethylcyclohexyl)acetic acid (14.8 g, 0.06 mol) was further added, followed by stirring for 14 hours. After the reaction, the solvent was distilled away, and ethyl acetate was added, followed by washing with water and a saturated aqueous sodium hydrogencarbonate solution and drying with magnesium sulfate. The residue was purified by silica-gel chromatography (ethyl acetate/hexane) to obtain a yellow oily substance (156 g).

### ¹H-NMR (200 MHz, chloroform-D)

δ ppm 0.82 - 1.88 (m, 6 H), 0.90,0.94(s, total 6H), 1.22,1.24(s, total 6H), 1.91 - 2.42 (m, 4 H), 3.60, 3.84 (s, total 3H ), 3.61,3.86(s, total 3H) , 3.68 - 3.96 (m, 1 H), 4.05 - 4.33 (m, 1 H), 5.20,5.22(s, total 2H), 5.39 - 5.49, 5.58 - 5.69 (m, total 1H), 6.44 (dd, J=8.79, 3.08 Hz, 1 H), 6.62 (d, J=2.64 Hz, 1 H), 6.78 (d, J=8.79 Hz, 1 H)

### Preparation Examples 1

**Lotion A :**

| (Formulation) | |
|---|---|
| 99.5% ethanol | 76.59 g |
| Benzyl alcohol | 0.4 g |
| Compound of the present invention | 1 g |
| Polyethylene glycol 400 | 16 g |
| Acetone | 6 g |
| 1-menthol | 0.01 g |

### (Preparation Method)

Polyethylene glycol 400, benzyl alcohol, and acetone were mixed, and the compound of the present invention was added to and dissolved into the mixture by stirring. 99.5% ethanol and 1-menthol were added to the mixture, followed by stirring to obtain a transparent lotion.

### Preparation Example 2

**Lotion B:**

| (Formulation) | |
|---|---|
| 99.5% ethanol | 70 g |
| Compound of the present invention | 1 g |
| Propylene glycol | 5 g |
| BHT (Dibutylhydroxytoluene) | 0.05 g |
| Purified water | 23.95 g |

### (Preparation Method)

The compound of the present invention, propylene glycol, and BHT were added to 99.5% ethanol, followed by stirring/dissolution. This solution and purified water were mixed to obtain a transparent lotion.

### Preparation Example 3

**Lotion C:**

| (Formulation) | |
|---|---|
| 99.5% ethanol | 79 g |
| Acetone | 11 g |
| Compound of the present invention | 5 g |
| 1,3-butylene glycol | 5 g |

### (Preparation Method)

The compound of the present invention and 1,3-butylene glycol were added to a mixture solution of 99.5% ethanol and acetone, followed by stirring/dissolution, thereby obtaining a transparent lotion.

### Preparation Example 4

**Lotion D (emulsion):**

| (Formulation) | |
|---|---|
| <Mixture Solution. A> | |
| Polysorbate 80 | 1 g |
| Sorbeth-40 tetraoleate | 1.5 g |
| Glyceryl Stearate | 1 g |
| Stearic acid | 0.5 g |
| Cetyl palmitate | 0.5 g |
| Compound of the present invention | 2 g |
| Squalane | 5 g |
| Methyl parahydroxybenzoate | 0.1 g |
| Propyl parahydroxybenzoate <Mixture Solution B> | 0.02 g |
| 1,3-butylene glycol | 5 g |
| Carboxyvinyl polymer | 0.1 g |
| Purified water | 83.28 g |

### (Preparation Method)

The mixture solution A and the mixture solution B were heated to 80°C. The mixture solution B was added to the mixture solution A, followed by cooling to 40°C, thereby obtaining an emulsion.

### Preparation Example 5

**Shampoo:**

| | |
|---|---|
| (Formulation) <Mixture Solution A> | |
| Triethanolamine lauryl sulfate Sodium lauroyl methylaminopropionate | 20 g |
| | 15 g |
| Cocamidepropyl betaine | 25 g |
| Isopropyl isostearate | 2 g |
| Compound of the present invention | 1 g |
| Ethyl aminobenzoate | 0.05 g |
| <Mixture Solution B> | |
| Hydrogenated soybean phospholipid | 0.2 g |
| 1,3-butylene glycol | 3 g |
| Purified water | 33.75 g |

### (Preparation Method)

The mixture solution A was heated to 70°C and added to the mixture solution B that was heated to 70°C. The mixed solution was stirred and cooled to 35°C, thereby obtaining a shampoo.

### Preparation Example 6

**Tablet:**

| (Formulation) | |
|---|---|
| Compound of the present invention | 2 g |
| Lactose | 12 g |
| Microcrystalline cellulose | 10.8 g |
| Low substituted hydroxypropylcellulose | |
| | 4 g |
| Hydroxypropylcellulose | 1 g |
| Magnesium stearate | 0.2 g |
| Purified water | 12 mg |

### (Preparation Method)

The above-listed components except for magnesium stearate were mixed in a mortar, and purified water was added, followed by granulation. The thus-obtained granule was dried, and magnesium stearate was added to the granule, followed by tabletting, thereby obtaining tablets each having a diameter of 8 mm and a weight of 200 mg.

### Test Example

Hair growth stimulatory effect measurement test in shaved mouse model:

### Method:

Dorsal hair of each of C57BL mice (female, about 7-week old) was shaved, and 200 µL of each of test compounds (90 mM) dissolved into 99% ethanol was applied to the shaved area once a day for 40 days from the day 3 after the shaving. As the evaluated compounds, the compound of the present invention and the compound (GPI-1511) disclosed in international Publication No. WO98/55090 were used. As a control, a group in which 200 µL of the vehicle was applied was used.

A hair growth state of the shaved area of each of 10 to 12 mice of each group was rated every 2 or 3 days after the start of administration by using the hollowing hair growth score criteria.

### (Hair growth score criteria)

1 = no hair growth
2 = hair growth in less than 30% of shaved area
3 = hair growth in 30% or more and less than 60% of shaved area
4 = hair growth in 60% or more and less than 90% of shaved area
5 = hair growth in 90% or more of shaved area

### Results:

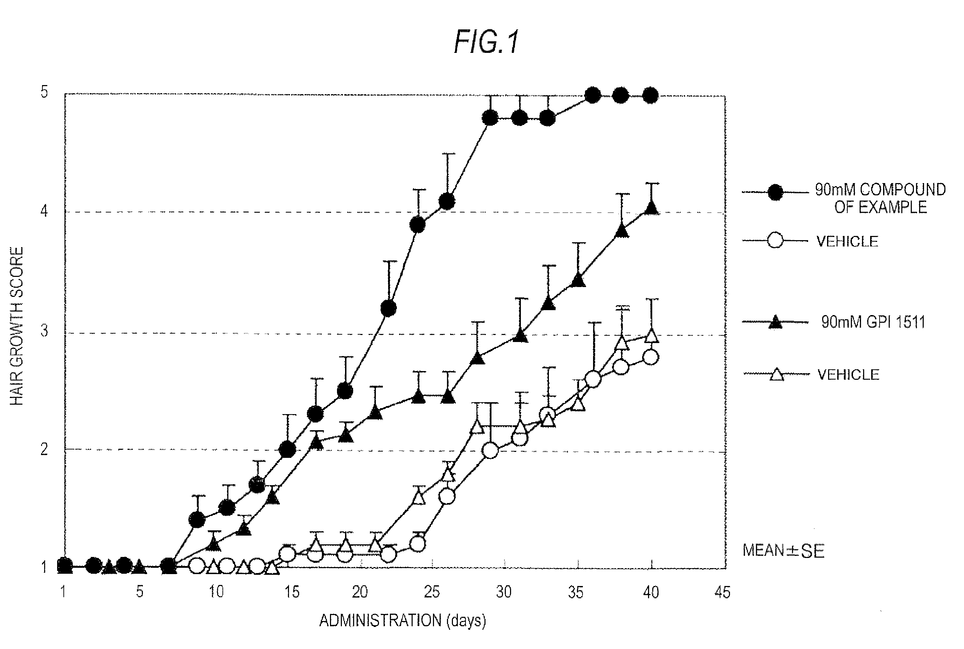

In the group where the compound of the present invention (18 µmol) or GPI-1511 (18 µmol) was administered, an increase in hair growth score was exhibited from an early stage as compared to the group in which the vehicle was administered (control group) (Fig. 1). The hair growth score of the group with the compound of the present invention was higher than that of the group with GPI-1511 over the whole test period. Particularly, from the day 19 of the administration, the increase in hair growth score in the group with the compound of the present invention is remarkable, thereby revealing that the compound achieves an excellent hair growth stimulatory effect. The hair growth stimulatory effect is exhibited by combination of a plurality of characteristics such as excellent stability, absorbability, tissue penetration property, and the like in addition to the rotamase inhibitory activity of the compound.

### Industrial Applicability

The prophylactic or therapeutic agent for alopecia containing the compound of the present invention as the active ingredient has the excellent hair restoration/pilatory action. Therefore, the agent is useful as a drug, a quasi-drug, or hair cosmetics.

### Brief Description of the Drawing

[Fig. 1] A diagram showing hair growth stimulatory effects of the compound of the present invention and GPI-1511 in shaved mouse model.

## Claims

1. A prophylactic or therapeutic agent for alopecia, comprising 1-[2-((2S)-2-{5-[(3,4-dimethoxyphenoxy)methyl]-1,2,4-ozadiazol-3-yl}pyrrolidin-1-yl-)-1,1-difluoro-2-oxoethyl]-3,3,5,5-tetramethylcyclohexanol as an active ingredient.

2. The prophylactic or therapeutic agent for alopecia according to claim 1, which is an external preparation.

3. The prophylactic or therapeutic agent for alopecia according to claim 1 or 2, wherein 0.00001 to 4 mg/cm² of 1-[2-((2S)-2-{5-[(3,4-dimethoxyphenoxy)methyl]-1,2,4-oxadiazol-3-yl}pyrrolidin-1-yl)-1,1-difluoro-2-oxoethyl]-3,3,5,5-tetramethylcyclohexanol is administered to a scalp.

4. A prophylactic or therapeutic method for alopecia, comprising administering 1-[2-((2S)-2-{5-[(3,4-dimethoxyphenoxy)methyl]-1,2,4-oxadiazol-3-yl}pyrrolidin-1-yl)-1,1-difluoro-2-oxoethyl]-3,3,5,5-tetramethylcyclohexanol in an amount effective for prophylaxis or treatment of alopecia to a mammal.

5. The prophylactic or therapeutic method for alopecia according to claim 4, wherein the mammal is a human.

6. The prophylactic or therapeutic method for alopecia according to claim 4 or 5, wherein the administration is performed by using an external preparation.

7. A use of 1-[2-((2S)-2-{5-[(3,4-dimethoxyphenoxy)methyl]-1,2,4-oxadiazol-3-yl}pyrrolidin-1-yl)-1,1-difluoro-2-oxoethyl]-3,3,5,5-tetramethylcyclohexanol for production of a prophylactic or therapeutic agent for alopecia.
